# EUROPEAN PATENT SPECIFICATION

(11) **EP 2 618 884 B1**
(45) Date of publication and mention of the grant of the patent: **28.08.2019**
(21) Application number: 11826488.6
(22) Date of filing: 21.09.2011
(51) Int. Cl.: A61M 25/06, A61M 5/32, A61M 5/00

(54) **NEEDLE TIP GUARD FOR INTRAVENOUS CATHETER ASSEMBLY**
NADELSPITZENFÜHRUNG FÜR EINE INTRAVENÖSE KATHETER-BAUGRUPPE
ÉLÉMENT DE PROTECTION DE POINTE D'AIGUILLE POUR ENSEMBLE CATHÉTER INTRAVEINEUX

(30) Priority: 21.09.2010 IN 2252DE2010
(43) Date of publication of application: 31.07.2013
(73) Proprietor: Poly Medicure Limited, Faridabad, Haryana 121004 (IN)
(72) Inventor: BAID, Rishi, New Delhi 110 048 (IN)
(74) Representative: Thum, Bernhard
(86) International application number: PCT/IB2011/054136
(87) International publication number: WO 2012/038899

(56) References cited:
- EP-A1- 1 132 103
- EP-A1- 2 016 963
- EP-A2- 0 747 085
- WO-A1-2013/021350
- WO-A2-2009/116080

## Description

### FIELD OF THE INVENTION

The present invention relates to a needle tip guard for medical device. More particularly, present invention relates to a needle tip guard for an intravenous catheter assembly.

### BACKGROUND OF THE INVENTION

Accidental needle sticks/pricks have become a major concern to health-care worker. It is well known and understood by healthcare workers that sharp surgical instruments, such as catheter needles, have a significant potential for harm to healthcare workers.During surgery, handling of these sharp instruments can lead to accidental sticks or puncture wounds exposing the healthcare worker to the infections such as AIDS and hepatitis. The chances of needle stick are increased during an emergency with several aspects require to be handled. Likewise, during disposal, an exposed needle point may be and usually is a threat to the medical waste handler.

Such needle tip guards are generally known and operative as a guard for the tip of a needle of the medical device by automatically covering the needle tip during withdrawal of the needle, for example, from a patient. The needle safety device thereby serves to prevent accidental pricking of, for example, a healthcare worker by the needle after removal of the needle from the medical device.

There is a need to protect the healthcare workers from accidental needle pricks. That being there is a constant need for having a needle tip guard to prevent accidental pricks and sticks.

EP 2 016 963 A1, which corresponds to the preamble of claim 1, discloses a needle safety device for a medical device, the needle safety device comprising: a base portion having a bore extending in an axial direction therethrough for receiving a needle; two opposing jaws extending from the base portion generally in the axial direction and each having a head portion in the region of its free end, wherein at least one of the head portions forms a locking shoulder for securing the needle safety device in a housing of the medical device; and an elastic element surrounding the jaws in a region between the base portion and the head portions, wherein the elastic element and the jaws are configured such that the jaws can be spread apart against a restoring force of the elastic element in order to allow the needle received in the bore to extend all the way through the needle safety device.

WO 2013/021350 A1, which is prior art according to Article 54(3) EPC, discloses a needle tip guard with two opposing jaws connected by two tension strips made from an elastic material, such as rubber, silicone or the like. The tension strips being fixed with their end portions to two opposite sides of the jaws, i.e. by using an adhesive or by molding these tensions strips integrally onto the jaws. The tension strips exert a restoring force on the jaws, when the jaws are spread apart by the needle. There is no additional elastic member surrounding the needle safety device, like a rubber ring or the like.

### SUMMARY OF THE INVENTION

The invention relates to a needle tip guard for a medical device, in particular for an intravenous catheter assembly according to claim 1, the needle tip guard comprising: a base portion having a bore extending along an axial direction for receiving a needle having a needle shaft such that the needle may extend through the bore and move relative to the needle tip guard from a spread-apart position in which the needle tip is outside the needle tip guard to a protective position in which the needle tip is covered by the needle tip guard; first and second jaw extending from the distal side of the base portion generally in the axial direction; each of the first and second jaw having a head portion in the region of its free end, wherein the first jaw rests over the needle shaft in the spread-apart position; one or more locking means onto the outer surface of the needle tip guard for securing the needle tip guard to the medical device; and at least one resilient member or elastic element integrally mounted onto the jaws in a region between the base portion and the head portions such that resilient member or elastic element exerts an inward restoring force on the jaws, when the jaws are fully or partially spread apart by the needle shaft.

Further features of the invention provides for the resilient member or elastic element integrally mounted to mounting features provided on each of the jaws. The mounting features may comprise one or more mounting projections and/or mounting recesses.

The resilient member or elastic element integrally mounted onto the jaws being adapted to form a partial sidewall such that together with the jaws it defines a chamber surrounding the needle in which the needle tip is held after complete withdrawal of the needle from the medical device once the jaws snaps together protecting the needle tip.

The integrated resilient member or elastic element comprises a ring like integrated form/structure fully surrounding the jaws.

Further features of the invention provides for one or more locking and/or releasing means provided either or both inside and/or outside of the catheter housing for engaging and/or releasing the needle guard in the form of one or more projections and/or recesses. Likewise, the outer surface of the needle tip guard may be provided with one or more locking and/or releasing means capable of being in a locking and/or releasing arrangement with the catheter housing of the medical device.

Another aspect of the invention relates to an intravenous catheter assembly, comprising a catheter tube, a catheter hub/housing, a needle having a needle tip, a needle shaft and a needle hub, and a needle tip guard as mentioned above slidably arranged on the needle for protecting the needle tip; and locking and/or releasing means formed inside and/or outside of the catheter hub/housing for engaging and/ or releasing the needle guard.

It is, therefore, one of the objects of this invention to provide a needle tip guard which provides a better protection against accidental needle pricks/sticks reducing the complexity and costs involved in the manufacture of prior known needle tip guards.

Another object of the present invention is to ensure low cost fabrication, the number of injection and/or insert molded parts required for a needle safety portion of a device based upon the invention may be as few as one.

Yet another object of the present invention is that the medical device such as catheter assembly are stored and shelved prior to use with the jaws spread apart over the needle. Due to the integrated resilient member or elastic element in the needle tip guard exerting its restoring force on the jaws in the spread apart state of the jaws, the elastic element ensures that the spread apart jaws will snap together and guard the needle tip upon withdrawn of the needle from the medical device even after a longer shelf life, thereby continuously ensuring a correct functioning of the needle tip guard.

### BRIEF DESCRIPTION OF THE DRAWINGS:

The present invention will now be explained in more detail in the following with reference to the preferred embodiments and to the accompanying drawings, wherein:
FIG. 1 illustrates a cross-section view of the needle tip guard according to the present invention;
FIG. 2 illustrates a side view of the needle tip guard according to the present invention;'
FIG. 3 illustrates a front view of the needle tip guard according to the present invention;
FIG. 4 illustrates a perspective view of the needle tip guard according to the present invention;
FIG. 5 illustrates a top view of the needle tip guard according to the present invention;
FIG. 6 illustrates a bottom view of the needle tip guard according to the present invention;
FIG. 7A illustrates a side of the needle tip region of the needle according to the present invention;
FIG. 7B illustrates a plan view region of the needle tip region of Fig. 7A according to the present invention;
FIG. 8 illustrates a plan view of the catheter housing of the medical according to the present invention;
FIG. 9A illustrates a cross-sectional view of a section of the catheter housing of Fig. 8 detailing an annular locking protrusion;
FIG. 9B illustrates cross-sectional view of a section of the catheter housing of Fig. 8 detailing an alternative embodiment of the annular locking protrusion.

### DETAILED DESCRIPTION OF THE INVENTION

In accordance with the present invention, an improved needle tip guard for a medical device is provided. While this invention is susceptible of embodiments in many different forms, there will be described herein specific embodiments with the understanding that the present disclosure is to be considered as an exemplification of the principles of the invention and is not intended to limit the invention to the embodiments detailed herein.

As used herein, the term 'proximal' refers to a region of the device or a location on the device which is closest to, for example, a clinician using the device. In contrast to this, the term 'distal' refers to a region of the device which is farthest from the clinician, for example, the distal region of a needle will be the region of a needle containing the needle tip which is to be inserted e.g. into a patient's vein. The term 'protective position' refers to the position in which the

With reference to accompanying figures, when the needle 24 extends all the way through the needle tip guard 10, such as prior to use of the intravenous catheter device (not shown), the needle tip guard 10 is slidably arranged partially and/or completely in a chamber of a medical device, such as in the catheter housing 42 of an intravenous catheter apparatus, and positioned near a proximal end of the needle hub. In this situation, the needle 34 extends completely through the needle guard 10 and the distal end section 24 of the first jaw 16 is supported on the needle shaft 38, whereby the first jaw 16 is forced away from the second jaw 18 from its relaxed position into a spread-apart position. The term 'spread-apart position' refers to the position in which the needle 34 having a needle shaft 38 passes through the jaws 16, 18 of the needle tip guard 10such that the needle tip 36 is outside the needle tip guard 10. As opposed to this the 'protective position' refers to the position in which the needle tip 36 is inside the needle tip guard 10 being covered by the jaws 16, 19 of the needle tip guard 10.

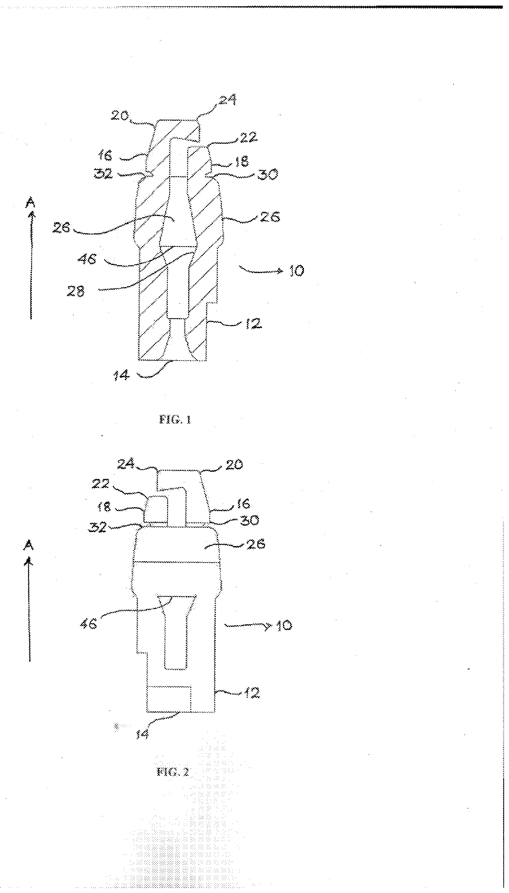

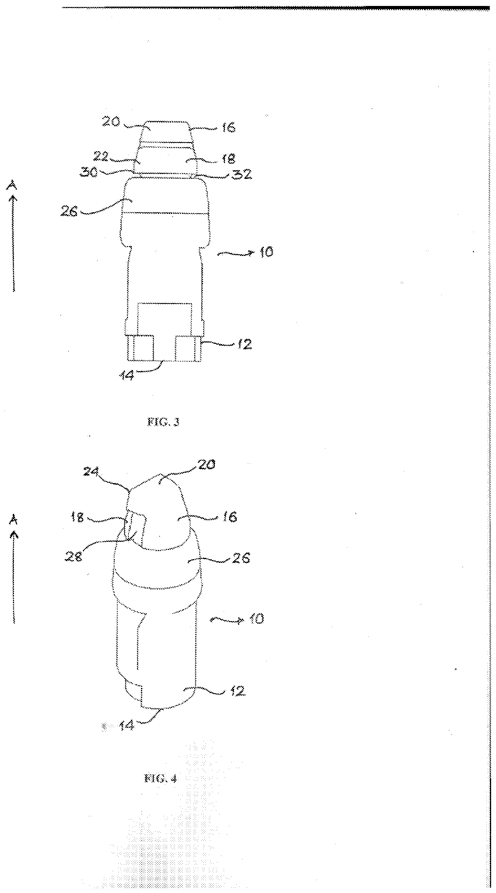

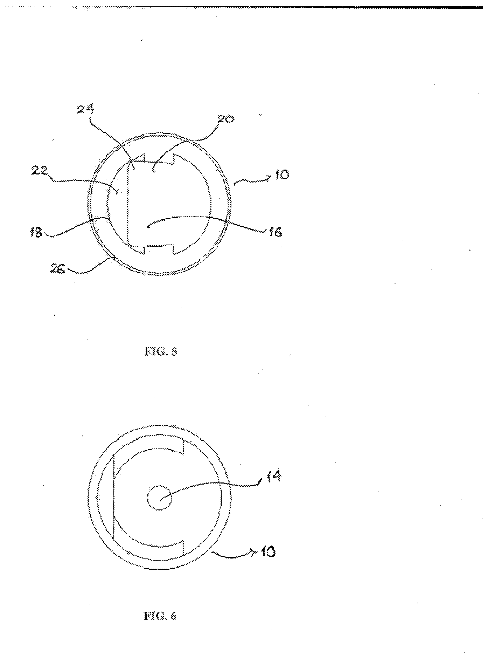

Referring to Figs. 1 to 6, the needle tip guard 10 comprises a base portion 12 which has a bore 14 extending in the axial direction - A therethrough for receiving the needle 34 having a needle shaft 38 thereby defining a passage for the needle 34. The dimension of the bore 14 is adapted to the shape and size of the principal outer profile of the needle 34 such that the needle 34 may extend through the bore 14 and move relative to the needle tip guard 10 to a protective position in which the needle tip 36 is covered by the needle guard 10.

As shown in Figs. 1 and 2, the needle tip guard 10 further includes first and second jaws 16, 18 extending distally from the base portion 12 generally in the axial direction A. The first jaw 16 has a first head portion 20 in the region of its free end, and the second jaw 18 has a second head portion 22 in the region of its free end. The first head portion 20 extends beyond the second head portion 22 and has a distal end section 24 at its free end, which extends towards the second jaw 18 in a direction generally perpendicular to the axial direction - A . The length of the distal end section 24 is as such that the end section 24 protrudes over at least a part of the second head portion 22, when the jaws 16, 18 are in a relaxed position as is shown in Figs. 2 to 6. Once the needle tip 36 is protected completely within the needle tip guard 10, the protruding distal end section 24 abuts the tip to extend beyond the first head portion 20 thereby securing the tip 36 of the needle 34. For ease in understanding, the relaxed position refers to a situation where no needle 34 extends through the needle tip guard 10.

As is illustrated in Figs. 1 to 4 the integrated resilient member or elastic element 26 covers a substantial part of the jaws 16, 18 in the axial direction - A. As shown the jaws 16, 18 are connected by at least one resilient member or elastic element 26 integrally mounted onto the jaws 16, 18 in a region between the base portion 12 and the head portions 20, 22. The resilient member or elastic element 26 exerts a restoring force on the jaws 16, 18, when the jaws 16, 18 are in spread-apart position allowing the needle 34 received in the bore 14 to extend all the way through the needle tip guard 10. As soon as the tip 36 of the needle 34 moves into the needle tip guard 10 i.e. in a position where the distal end section 24 of the first jaw 16 no longer rests over the needle shaft 38, the first jaw 16 snaps back into its relaxed position due to the restoring force exerted by the integrated resilient member or elastic element 26. The resilient member or elastic element 26 integrally mounted onto the jaws is adapted to form a partial sidewall such that together with the jaws 16, 18 it defines a chamber 46 surrounding the needle 34 in which the needle tip 36 is held after complete withdrawal of the needle 34 from the medical device once the jaws 16, 18 snaps together protecting the needle tip 36.

As can be seen in Fig 1, the dimension of the inner portion 28 of the bore in the region adjacent the region of integrally mounted resilient member or elastic element 26 is larger than the dimension inner portion 28 in the region of proximal and distal section of the bore 14. The larger dimension of the inner portion 28 of the bore 14 provides a drawing force for the jaws 16, 18 resulting in improving the restoring force to be exerted by said jaws 16, 18. It is to be noted, however, that the dimension of the inner portion 28 of the bore 14 can have any other suitable dimension or can be uniform through-out extending generally in an axial direction - A.

The jaws 16, 18 are slightly angled towards each other in the region of the resilient member or elastic element 26, resulting in the outer profile of the needle tip guard 10 being tapered towards the distal head portions 20, 22. The outer surface of the jaws 16, 18 may comprises one or more annular protrusions being formed therein. It is to be noted, however, that the needle tip guard can be made without having such annular protrusions.

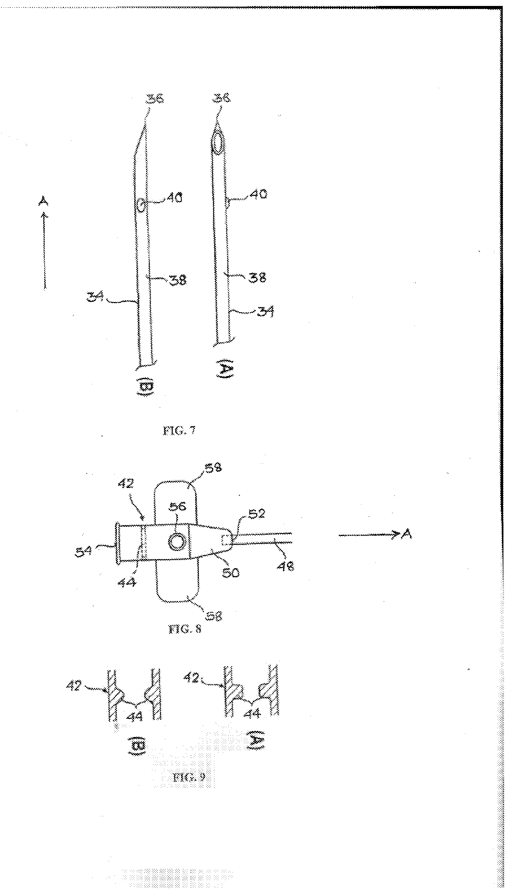

Further, a locking shoulder 30 is formed at the side of each head portions 20, 22 forming a recess or groove 32 adjacent the distal region of integral resilient member or elastic element 26. One or more locking means are provided in the inner surface of the catheter housing 42 such as an annular locking projection 44 as shown in Figs. 8 and 9. The annular locking projection 44 may have a profile with rounded edges or substantially rectangular edges or any other profile suitable for locking of the needle tip guard 10 in the catheter housing 42. Further, such locking projection 44 may have continuous annular shape or may have one or more interruptions in between. The housing 42 comprises a main body 50 of generally tubular form and extending in an axial direction - A. The main body has a distal end 52 and a proximal end 54. A catheter 48 is attached to the main body 50 at the distal end 52 of the main body 50. A port 56 extends from the main body 50 in a direction generally perpendicular to the axial direction A. Wings 58 are provided at the main body 50 opposite from the port 56. The main body 50 defines a chamber forming the housing 42 extending from the proximal end 54 towards the distal end 52.

When the needle tip guard 10 is positioned in the catheter housing allowing the needle 34 to extend all the way through the needle tip guard 10 and the catheter housing, the annular locking projection 44 is received in the recess 32 defined by the locking shoulder 30 in the spread-apart position thereby securing the needle tip guard 10 in the catheter housing 42. Preferably, in such a situation, the needle tip guard 10 cannot be pulled out against or relative to the movement of the needle 34 body since the locking shoulder 30 engages with the annular locking projection 44 provided in the catheter housing 42. Thus, as long as the jaws 16, 18 are in a spread-apart position, the needle tip guard 10 is secured against the axial movement relative to the needle 34 body of the catheter device, i.e. the needle tip guard 10 remains steadily in the catheter housing 42. However, when the first jaw 16 adopts its relaxed position as discussed above, the locking protrusion is released from the recess 32. As a result, the needle tip guard 10 is free to be moved protecting the tip 36 of the needle 34.

Further, it is to be noted that one or more locking and/or releasing means may be provided both inside and/or outside of the catheter housing 42 for engaging and/or releasing the needle guard 10 (Fig. 9). Likewise, the outer surface of the needle tip guard 10 may be provided with one or more locking and/or releasing means capable of being in a locking and/or releasing arrangement with the catheter housing 42. For example, the base portion 12 of the needle tip guard 10 may be provided with one or more arms (not shown) having locking means for example locking protrusions at distal ends of either of said arms and/or both the arms to interact with a corresponding recess or projection formed at the outside surface of the catheter housing 42.

As can be seen in Fig 7, the needle 34 has a needle tip 36 and a needle shaft 38. The needle shaft 38 has a principal outer profile and defines an axial direction - A. The needle 34 near the tip 36 of the needle is provided with an enlargement 40. The maximum outer dimension/profile of the enlargement 40 is greater than the principal outer dimension/profile of the needle shaft 38. The position of the enlargement 40 formed on the outer surface of the needle shaft 38 is selected such that the enlargement 40 abuts the base portion 12 of the needle tip guard 10 as soon as first jaw 16 of the needle tip guard 10 no longer rests over the needle shaft 38 in the spread-apart position. Since the maximum outer dimension/profile of the needle 34 in the region of the enlargement 40 is larger than the dimension of the bore 14 in the base portion 12, the needle 34 cannot be further pulled out of the needle tip guard 10. More specifically, because of the enlargement 40, the needle tip guard 10 cannot slide off the needle 34 during normal use once the needle guard 10 is in a protective position protecting the needle tip 36, unless an excessive external force is applied to the needle 34 and/or needle tip guard 10.

The enlargement 40, for example, can be formed as a crimp by crimping of the needle shaft 38 or as bump with or without addition of additional material by subjecting the needle 34 to concentrated heat at a localized area, for example, using a welding process or a laser welding process. However, it is also possible to form the enlargement 40 by dispensing additional material onto the outer surface of the needle shaft 38, for example, by welding, soldering or by use of adhesive or glue. Although, only one enlargement 40 is shown in Figs, it is to be noted that more than one enlargement 40 may be present on the outer surface of the needle shaft 38.

Thus, the needle tip guard 10 with the integrated resilient member or elastic element 26 prevents the jaws 16, 18 of the needle tip guard 10 from becoming loose when the needle tip guard 10 slides along the needle 34, thereby aiding and ensuring a correct functioning of the needle tip guard 10. Further, the integrated resilient member or elastic element 26 prevents the needle tip 36 protruding and/or slipping sideways out of the needle tip guard 10, thereby increasing the protection function of the needle tip guard 10.

The integrated resilient member or elastic element 26 is made from a resilient material or elastic material having elastic properties such as, not being limited to, rubber, silicone or the like. Due to its elastic properties, the resilient member or elastic element or ring creates a restoring force on the jaws 16, 18, when the needle 34 fully extends through the needle tip guard 10 thereby spreading the jaws 16, 18 apart and expanding the resilient member or elastic element 26. As soon as the needle tip 36 distal end section 24 of the first jaw 16 upon pulling the needle 34 through the needle safety device 10, the jaws 16, 18 are positively collapsed by the integrated resilient member or elastic element 26 due to its restoring force securing the tip 36 of the needle 34.

For the purpose of a simplified and cost-effective production of the needle tip guard 10, the integrated resilient member or elastic element 26 is formed integrally onto the jaws, for example, by injection and/or insert moulding. The resilient member or elastic element 26 may also be configured integrally onto the jaws 16, 18 by other suitable methods such as by use of an adhesive or glue. Further, for a simplified and cost-effective production of the needle tip guard 10, the base portion 12, the resilient member or elastic element 26 and the jaws 16, 18 may be formed from a plastic material, for example, by injection moulding. Alternatively, the base portion 12 and the jaws 16, 18 may be formed from a metal material or combination of materials, such as a different plastic material, a different metal material or a different combination of plastic and/or metal materials. For example, one of the jaws 16, 18 may be made of a metal material and other one 16, 18 may be made of plastic material. Likewise, the base portion 12 may be made from a metal material and the jaws 16, 18 may be made from a plastic material or vice versa. It is also to be noted that the inner part of the jaws 16, 18, which contacts the needle shaft 38, is made from a thermoplastic material such as TPE, whereas the outer part of the jaws 16, 18 may be made from a different material, for example, a plastic, metal, composite or elastomer material, so that the needle tip guard 10 causes less friction when sliding along the needle thereby facilitating the withdrawal of the needle 34.

In various embodiments of the present invention the integrated resilient member or elastic element 26 may comprise a ring like integrated form/structure partially or fully surrounding the jaws 16, 18, and/or clamp, bracket, 'C' clip or the like surrounding the jaws 16, 18 only in part.

Although, the invention has been described with reference to certain specific embodiments and examples, it would be appreciated by those skilled in the art that the invention may be embodied in many forms without departing from the scope of the invention as set forth in the invention. Thus, variations of preferred embodiments as disclosed may become apparent to those of ordinary skill in the art upon reading the foregoing description.

Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context. The specification and drawings, therefore, are to be regarded in an illustrative rather than a restrictive manner without departing from the scope of the invention, the invention resides in the claims hereinafter appended.

## Claims

1. A needle tip guard (10) for a medical device, in particular for an intravenous catheter assembly, the needle tip guard (10) comprising:
a base portion (12) having a bore (14) extending along an axial direction (A) for receiving a needle (34) having a needle shaft (38) such that the needle (34) may extend through the bore (14) and move relative to the needle tip guard (10) from a spread-apart position in which the needle tip (36) is outside the needle tip guard (10) to a protective position in which the needle tip (36) is covered by the needle tip guard (10);
first (16) and second (18) jaw extending from the distal side of the base portion (12) generally in the axial direction (A);
each of the first (16) and second (18) jaw having a head portion (20, 22) in the region of its free end, wherein the first jaw (16) rests over the needle shaft (38) in the spread-apart position;
one or more locking means onto the outer surface of the needle tip guard (10) for securing the needle tip guard (10) to the medical device; and
at least one resilient member or elastic element (26) on the jaws (16, 18) in a region between the base portion (12) and the head portions (20, 22) such that said resilient member or elastic element (26) exerts an inward restoring force on the jaws (16, 18), when the jaws (16, 18) are fully or partially spread apart by the needle shaft (38), wherein said resilient member or elastic element (26) forms a partial sidewall such that together with the jaws (16, 18) it defines a chamber (46) and is made from a material having elastic properties and has a ring like integrated form/structure fully surrounding the jaws (16, 18);
**characterized in that**
the at least one resilient member or elastic element (26) is formed integrally onto the jaws (16, 18).

2. The needle tip guard (10) according to claim 1, wherein the locking means comprise one or more locking shoulder (30) forming a recess or groove (32).

3. The needle tip guard (10) according to claim 1, wherein the bore (14) has a dimension being adapted to the shape and size of the principal outer profile of the needle (34) such that the needle (34) may extend through the bore (14) and move relative to the needle tip guard (10) to a protective position in which the needle tip (36) is covered by the said needle guard (10).

4. The needle tip guard (10) according to claim 1, wherein the first head portion (20) has distal end section (24) at its free end which extends beyond the second head portion (22) and the said distal end section (24) thereof extends towards the said second jaw (18) generally perpendicular to the axial direction (A).

5. The needle tip guard (10) according to claim 4, wherein the length of the distal end section (24) is such that the end section (24) protrudes over at least a part of the second head portion (22).

6. The needle tip guard (10) according to claim 1, wherein the resilient member or elastic element (26) is mounted to mounting features comprising mounting projections and or mounting recesses or the like provided on each of the jaws (16, 18).

7. An intravenous catheter assembly comprising: a catheter tube (48), a catheter hub/housing (42) defining a chamber, a needle (34) having a needle tip (36) at the distal end, a needle shaft (38) and a needle hub, and a needle tip guard (10) in particular according to any one of the claims 1 to 6 slidably arranged on the needle (34) for protecting the needle tip (36) which is housed partially and/or completely in the catheter housing (42) such that the distal end section (24) of the first jaw (16) rests over the needle shaft (38) in the spread-apart position.

8. The intravenous catheter assembly according to claim 7, wherein the inner and/or outer surface of the catheter housing (42) comprises one or more locking and/or releasing means in the form of one or more projections (44) and/or recesses.

9. The needle tip guard (10) according to any one of preceding claims 1 to 6, wherein said chamber (46) surrounds the needle (34) such that the needle tip (36) is held in said chamber (46) after complete withdrawal of the needle (34) from the medical device once the jaws (16, 18) snap together protecting the needle tip (36).

10. The needle tip guard (10) according to any one of preceding claims 1-6 or 9, wherein the dimension of an inner portion (28) of the bore (14) in the region adjacent the region of integrally mounted resilient member or elastic element (26) is larger than the dimension of the inner portion (28) in the region of a proximal and distal section of the bore (14).

11. The needle tip guard (10) according to any one of preceding claims 1 to 6, 9 or 10, wherein the jaws (16, 18) are slightly angled towards each other in the region of the resilient member or elastic element (26), such that the outer profile of the needle tip guard (10) is tapered towards the head portions (20, 22).

12. The intravenous catheter assembly (10) according to claim 7, wherein one or more locking and/or releasing means (44) may be provided both inside and/or outside of the catheter housing (42) for engaging and/or releasing the needle guard (10).

13. The intravenous catheter assembly (10) according to claim 7, 8 or 12, wherein the outer surface of the needle tip guard (10) is provided with one or more locking and/or releasing means capable of being in a locking and/or releasing arrangement with the catheter housing (42).

## Patentansprüche

1. Nadelspitzenschutz (10) für ein medizinisches Gerät, insbesondere für eine intravenöse Katheteranordnung, wobei der Nadelspitzenschutz (10) umfasst:
einen Basisabschnitt (12) mit einer Bohrung (14), die sich entlang einer Axialrichtung (A) erstreckt, um eine Nadel (34) mit einem Nadelschaft (38) aufzunehmen, sodass sich die Nadel (34) durch die Bohrung (14) erstrecken und sich relativ zum Nadelspitzenschutz (10) von einer auseinandergespreizten Position, in der sich die Nadelspitze (36) außerhalb des Nadelspitzenschutzes (10) befindet, in eine Schutzposition bewegen kann, in der die Nadelspitze (36) von dem Nadelspitzenschutz (10) bedeckt ist;
eine erste (16) und zweite (18) Backe, die sich von der distalen Seite des Basisabschnitts (12) allgemein in der axialen Richtung (A) erstrecken;
wobei jede der ersten (16) und zweiten (18) Backen im Bereich ihres freien Endes einen Kopfabschnitt (20, 22) aufweist, wobei die erste Backe (16) in der auseinandergespreizten Position über dem Nadelschaft (38) aufliegt;
ein oder mehrere Verriegelungsmittel an der Außenfläche des Nadelspitzenschutzes (10) zum Sichern des Nadelspitzenschutzes (10) an dem medizinischen Gerät; und
mindestens ein nachgiebiges Teil oder elastisches Element (26) an den Backen (16, 18) in einem Bereich zwischen dem Basisabschnitt (12) und den Kopfabschnitten (20, 22), sodass das nachgiebige Teil oder elastische Element (26) eine nach innen gerichtete Rückstellkraft auf die Backen (16, 18) ausübt, wenn die Backen (16, 18) durch den Nadelschaft (38) ganz oder teilweise auseinandergespreizt sind, wobei das elastische Teil oder elastische Element (26) eine Teilseitenwand bildet, sodass es zusammen mit den Backen (16, 18) eine Kammer (46) definiert und aus einem Material hergestellt ist, das elastischen Eigenschaften aufweist, und eine ringartig integrierte Form/Struktur aufweist, die die Backen (16, 18) vollständig umgibt;
**dadurch gekennzeichnet, dass**
das mindestens eine nachgiebige Teil oder elastische Element (26) einstückig auf den Backen (16, 18) ausgebildet ist.

2. Nadelspitzenschutz (10) nach Anspruch 1, wobei die Verriegelungsmittel eine oder mehrere Verriegelungsschultern (30) aufweisen, die eine Vertiefung oder Nut (32) bilden.

3. Nadelspitzenschutz (10) nach Anspruch 1, wobei die Bohrung (14) eine Abmessung aufweist, die an die Form und Größe des Hauptaußenprofils der Nadel (34) angepasst ist, sodass sich die Nadel (34) durch die Bohrung erstrecken (14) und sich relativ zum Nadelspitzenschutz (10) in eine Schutzposition bewegen kann, in der die Nadelspitze (36) von dem Nadelspitzenschutze(10) abgedeckt ist.

4. Nadelspitzenschutz (10) nach Anspruch 1, wobei der erste Kopfabschnitt (20) an seinem freien Ende einen distalen Endteilabschnitt (24) aufweist, der sich über den zweiten Kopfabschnitt (22) hinaus erstreckt, und sich dessen distaler Endteilabschnitt (24) in Richtung der zweiten Backe (18) allgemein senkrecht zu der axialen Richtung (A) erstreckt.

5. Nadelspitzenschutz (10) nach Anspruch 4, wobei die Länge des distalen Endteilabschnitts (24) derart ist, dass der Endteilabschnitt (24) über mindestens einen Teil des zweiten Kopfabschnitts (22) vorsteht.

6. Nadelspitzenschutz (10) nach Anspruch 1, wobei das nachgiebige Teil oder elastische Element (26) an Anbringungsmerkmalen angebracht ist, die Anbringungsvorsprünge und/oder Anbringungsvertiefungen oder dergleichen aufweisen, die an jeder der Backen (16, 18) vorgesehen sind.

7. Intravenöse Katheteranordnung, aufweisend: ein Katheterrohr (48), eine Katheternabe/ein Kathetergehäuse (42), die/das eine Kammer definiert, eine Nadel (34), die eine Nadelspitze (36) am distalen Ende aufweist, einen Nadelschaft (38) und einen Nadelnabe, und einen Nadelspitzenschutz (10), insbesondere nach irgendeinem der Ansprüche 1 bis 6, der verschiebbar an der Nadel (34) angeordnet ist, um die Nadelspitze (36) zu schützen, die teilweise und/oder vollständig in dem Kathetergehäuse (42) untergebracht ist, sodass der distale Endteilabschnitt (24) der ersten Backe (16) in der auseinandergespreizten Position über dem Nadelschaft (38) aufliegt.

8. Intravenöse Katheteranordnung nach Anspruch 7, wobei die Innen- und/oder Außenfläche des Kathetergehäuses (42) ein oder mehrere Verriegelungs- und/oder Freigabemittel in der Form eines oder mehrerer Vorsprünge (44) und/oder Vertiefungen aufweist.

9. Nadelspitzenschutz (10) nach irgendeinem der vorhergehenden Ansprüche 1 bis 6, wobei die Kammer (46) die Nadel (34) derart umgibt, dass die Nadelspitze (36) in der Kammer (46) gehalten wird, nachdem die Nadel (34) vollständig aus dem medizinischen Gerät gezogen ist, sobald die Backen (16, 18) zusammenschnappen, wobei sie die Nadelspitze (36) schützen.

10. Nadelspitzenschutz (10) nach irgendeinem der vorhergehenden Ansprüche 1 bis 6 oder 9, wobei die Abmessung eines inneren Abschnitts (28) der Bohrung (14) im Bereich neben dem Bereich des einstückig gelagerten nachgiebigen Teils oder elastischen Elements (26) größer ist als die Abmessung des inneren Abschnitts (28) in dem Bereich eines proximalen und distalen Teilabschnitts der Bohrung (14).

11. Nadelspitzenschutz (10) nach irgendeinem der vorhergehenden Ansprüche 1 bis 6, 9 oder 10, wobei die Backen (16, 18) in dem Bereich des nachgiebigen Teils oder elastischen Elements (26) leicht zueinander abgewinkelt sind, sodass sich das Außenprofil des Nadelspitzenschutzes (10) zu den Kopfabschnitten (20, 22) hin verjüngt.

12. Intravenöse Katheteranordnung (10) nach Anspruch 7, wobei ein oder mehrere Verriegelungs- und/oder Freigabemittel (44) sowohl innerhalb als auch/oder außerhalb des Kathetergehäuses (42) zum Eingreifen und/oder Freigeben des Nadelschutzes (10) vorgesehen sein können.

13. Intravenöse Katheteranordnung (10) nach Anspruch 7, 8 oder 12, wobei die Außenfläche des Nadelspitzenschutzes (10) mit einem oder mehreren Verriegelungs- und/oder Freigabemitteln versehen ist, die sich in einer Verriegelungs- und/oder Freigabeanordnung mit dem Kathetergehäuse (42) befinden können.

## Revendications

1. Garde-pointe d'aiguille (10) pour un dispositif médical, en particulier pour un ensemble de cathéter intraveineux, le garde-pointe d'aiguille (10) comprenant :
une partie de base (12) ayant un alésage (14) s'étendant le long d'un sens axial (A) pour recevoir une aiguille (34) ayant une tige (38) d'aiguille de telle manière que l'aiguille (34) peut s'étendre à travers l'alésage (14) et se déplacer par rapport au garde-pointe d'aiguille (10) d'une position espacée dans laquelle la pointe (36) d'aiguille est à l'extérieur du garde-pointe d'aiguille (10) à une position de protection dans laquelle la pointe (36) d'aiguille est recouverte par le garde-pointe d'aiguille (10) ;
une première (16) et une deuxième (18) mâchoire s'étendant depuis le côté distal de la partie de base (12) de façon générale dans le sens axial (A) ;
chacune de la première (16) et de la deuxième (18) mâchoire ayant une partie de tête (20, 22) dans la région de son extrémité libre, dans lequel la première mâchoire (16) repose par-dessus la tige (38) d'aiguille dans la position espacée ;
un ou plusieurs moyens de verrouillage sur la surface extérieure du garde-pointe d'aiguille (10) pour sécuriser le garde-pointe d'aiguille (10) sur le dispositif médical ; et
au moins un élément résilient ou élément élastique (26) sur les mâchoires (16, 18) dans une région entre la partie de base (12) et les parties de tête (20, 22) de telle manière que ledit élément résilient ou élément élastique (26) exerce une force de restauration vers l'intérieur sur les mâchoires (16, 18), lorsque les mâchoires (16, 18) sont totalement ou partiellement espacées par la tige (38) d'aiguille, dans lequel ledit élément résilient ou élément élastique (26) forme une paroi latérale partielle de telle manière qu'ensemble avec les mâchoires (16, 18) il définit une chambre (46) et est constitué d'un matériau ayant des propriétés élastiques et a une forme/structure intégrée de type bague entourant totalement les mâchoires (16, 18) ;
**caractérisé en ce que**
l'au moins un élément résilient ou élément élastique (26) est formé de façon intégrale sur les mâchoires (16, 18).

2. Garde-pointe d'aiguille (10) selon la revendication 1, dans lequel le moyen de verrouillage comprend un ou plusieurs épaulements de verrouillage (30) formant un renfoncement ou une rainure (32).

3. Garde-pointe d'aiguille (10) selon la revendication 1, dans lequel l'alésage (14) a une dimension étant adaptée à la forme et à la taille du profil extérieur principal de l'aiguille (34) de telle manière que l'aiguille (34) peut s'étendre à travers l'alésage (14) et se déplacer par rapport au garde-pointe d'aiguille (10) jusqu'à une position de protection dans laquelle la pointe (36) d'aiguille est recouverte par ledit garde-aiguille (10).

4. Garde-pointe d'aiguille (10) selon la revendication 1, dans lequel la première partie de tête (20) a une section d'extrémité distale (24) à son extrémité libre qui s'étend au-delà de la deuxième partie de tête (22) et ladite section d'extrémité distale (24) de celle-ci s'étend vers ladite deuxième mâchoire (18) de façon généralement perpendiculaire au sens axial (A).

5. Garde-pointe d'aiguille (10) selon la revendication 4, dans lequel la longueur de la section d'extrémité distale (24) est telle que la section d'extrémité (24) fait saillie par-dessus au moins une partie de la deuxième partie de tête (22).

6. Garde-pointe d'aiguille (10) selon la revendication 1, dans lequel l'élément résilient ou élément élastique (26) est monté sur des caractéristiques de montage comprenant des projections de montage et/ou des renfoncements de montage ou similaire prévus sur chacune des mâchoires (16, 18).

7. Ensemble de cathéter intraveineux comprenant : un tube (48) de cathéter, un raccord/logement (42) de cathéter définissant une chambre, une aiguille (34) ayant une pointe (36) d'aiguille à l'extrémité distale, une tige (38) d'aiguille et un raccord d'aiguille, et un garde-pointe d'aiguille (10) en particulier selon l'une quelconque des revendications 1 à 6 agencé coulissant sur l'aiguille (34) pour protéger la pointe (36) d'aiguille qui est logée partiellement et/ou complètement dans le logement (42) de cathéter de telle manière que la section d'extrémité distale (24) de la première mâchoire (16) repose par-dessus la tige (38) d'aiguille dans la position espacée.

8. Ensemble de cathéter intraveineux selon la revendication 7, dans lequel la surface intérieure et/ou extérieure du logement (42) de cathéter comprend un ou plusieurs moyens de verrouillage et/ou de libération sous la forme d'un(e) ou plusieurs projections (44) et/ou renfoncements.

9. Garde-pointe d'aiguille (10) selon l'une quelconque des revendications précédentes 1 à 6, dans lequel ladite chambre (46) entoure l'aiguille (34) de telle manière que la pointe (36) d'aiguille est maintenue dans ladite chambre (46) après retrait complet de l'aiguille (34) du dispositif médical une fois que les mâchoires (16, 18) s'enclenchent ensemble protégeant la pointe (36) d'aiguille.

10. Garde-pointe d'aiguille (10) selon l'une quelconque des revendications précédentes 1 à 6 ou 9, dans lequel la dimension d'une partie intérieure (28) de l'alésage (14) dans la région adjacente à la région d'élément résilient ou élément élastique (26) monté de façon intégrale est plus grande que la dimension de la partie intérieure (28) dans la région d'une section proximale et distale de l'alésage (14).

11. Garde-pointe d'aiguille (10) selon l'une quelconque des revendications précédentes 1 à 6, 9 ou 10, dans lequel les mâchoires (16, 18) sont légèrement en angle l'une vers l'autre dans la région de l'élément résilient ou élément élastique (26), de telle manière que le profil extérieur du garde-pointe d'aiguille (10) s'effile vers les parties de tête (20, 22).

12. Ensemble (10) de cathéter intraveineux selon la revendication 7, dans lequel un ou plusieurs moyens de verrouillage et/ou de libération (44) peuvent être prévus à la fois à l'intérieur et/ou à l'extérieur du logement (42) de cathéter pour engager et/ou libérer le garde-aiguille (10).

13. Ensemble (10) de cathéter intraveineux selon la revendication 7, 8 ou 12, dans lequel la surface extérieure du garde-pointe d'aiguille (10) est prévue avec un ou plusieurs moyens de verrouillage et/ou de libération aptes à être dans un agencement de verrouillage et/ou de libération avec le logement (42) de cathéter.
